# EUROPEAN PATENT SPECIFICATION

(11) **EP 1 378 242 B1**
(45) Date of publication and mention of the grant of the patent: **27.12.2006**
(21) Application number: 03013792.1
(22) Date of filing: 18.06.2003
(51) Int. Cl.: A61K 35/14

(54) **Methods and compositions for the stabilization of brain natriuretic peptide (BNP) in blood samples**
Verfahren und Zusammensetzungen zur Stabilisierung des Brain-Natriuretic-Peptids (BNP) in Blutproben
Méthodes et compositions destinées à la stabilisation du Brain Natriuretic Peptide (BNP) dans des prises de sang

(30) Priority: 19.06.2002 US 389991 P
(43) Date of publication of application: 07.01.2004
(73) Proprietor: BAYER CORPORATION, Pittsburgh, PA 15205 (US)
(72) Inventor: Belensky. Alexander, New York, N.Y-10471 (US); Bluestein, Barry, Mansfiled, Mass. 02048 (US)
(74) Representative: Burkert, Frank

(56) References cited:
- EP-A- 1 030 177
- WO-A-93/07489
- WO-A-02/074234
- US-A- 5 444 041
- DATABASE WPI Section Ch, Week 199332 Derwent Publications Ltd., London, GB; Class B04, AN 1993-252670 XP002253612 "Peptide composition having long term stability - comprises freeze-dried solution containing calcitonin or natriuresis peptide and sucrose and/or maltose stabiliser" & JP 05 170664 A (SUNTORY LTD), 9 July 1993 (1993-07-09)

## Description

The present invention relates to method, compositions and kits for reducing the proteolytic degradation of brain natriuretic peptide (BNP), thereby stabilizing this peptide, in blood based samples, such as plasma and serum.

### BACKGROUND OF THE INVENTION

Brain natriuretic peptide (BNP) is a specific and sensitive indicator of congestive heart failure. BNP is a vasoreactive cardiac peptide hormone which is synthesized and secreted into the bloodstream primarily from the heart ventricles. (H. Shimizu et al., 2001, *Clinica Chimica Acta,* 305:181-186; H. Shimizu et al., 1999, *Clinica Chimica Acta,* 285:169-172). This peptide hormone promotes natriuresis and diuresis, acts as a vasodilator and antagonizes the vasoconstrictor effects of renin-angiotensin-aldosterone system. (A. Goginet-Georges et al., 2000, *Clin. Chem. Lab. Med.,* 38(6):519-523).

Because plasma concentrations of BNP increase with the decline of heart function, particularly ventricular function, the measurement of the BNP concentration in blood is useful for the diagnosis and prognosis of acute myocardial infarction (AMI) or heart failure. (E. Morita et al., 1993, *J*. *Am. Coll. Cardiol.,* 88:82-91; T. Tsutamoto et al., 1989, *Am. Heart J.,* 117:599-606; M. Mukoyama et al., 1990, *Lancet,* 335:801-802; T.A. McDonagh et al., 1998, *Lancet,* 351:9-13; T. Omland et al., 1996, *Circulation,* 93:1963-1969; T. Tsutamoto et al., 1997, *Circulation,* 96:509-516). Since heart disease and heart failure are major health problems worldwide, BNP has been proposed as a biochemical marker for use in screening patients to select for further cardiac investigations and/or treatment. (A. Goginet-Georges et al., 2000, *Clin. Chem. Lab. Med.,* 38(6):519-523).

Application of BNP as a diagnostic marker for identifying patients with heart disease, e.g., left ventricular systolic dysfunction, is complicated by the peptide's poor stability in blood samples. (A. Goginet-Georges et al., 2000, *Clin. Chem. Lab. Med.,* 38(6):519-523; H. Shimizu et al., 2001, *Clinica Chimica Acta*, 305:181-186; H. Shimizu et al., 1999, *Clinica Chimica Acta*, 285:169-172; D.R. Murdoch et al., 1999, *Heart,* 81:212-213; T. Tsuju et al., 1994, *Clin. Chem.*, 40(4):672-673). Both endogenous BNP and synthetic forms of the peptide are prone to relatively rapid decomposition in blood plasma and serum as a result of proteolysis. In addition, rapid degradation of BNP occurs after separation of plasma from whole blood. This degradation progresses even on storage under refrigerated conditions, thus making it difficult to accurately measure BNP in anything other than fresh blood samples, unless the fresh sample is immediately frozen.

Although approaches for stabilizing BNP in blood samples have been reported in the art, none are completely satisfactory, and most are inefficient. For example, attempts to stabilize BNP in patient blood samples have involved the use of collection tubes of a particular composition (H. Shimizu et al., 1999, *Clinica Chimica Acta*, 285:169-172); the addition of EDTA to blood samples (D.R. Murdoch et al., 1999, *Heart,* 81:212-213); and the addition of a combination of aprotinin and benzamidine to blood samples (T. Tsuju et al., 1994, *Clin. Chem.,* 40(4):672-673).

The problem of preventing or significantly reducing BNP degradation in blood samples is still a viable one in the area of hematology and better methods and solutions for stabilizing BNP are needed. The present invention provides a solution to the existing difficulty of stabilizing BNP so as to reduce or prevent its proteolytic degradation in blood samples.

### DESCRIPTION OF THE FIGURES

**FIG. 1** shows the effect of inhibitors on reducing the degradation of native (endogenous) BNP in a patient sample stored at 4°C over time. In these studies, PPACK was used at a concentration of 35 µg/ml and leupeptin was used at a concentration of 50 µg/ml. For the sample containing inhibitors, significantly more BNP was present at about 25 hours of storage at 4°C compared with the sample containing no inhibitors.

**FIG. 2** shows the effect of inhibitors on reducing the degradation of exogenous synthetic BNP (10,000 pg/ml) spiked into a human plasma pool (Intergen, Milford, MA). BNP in human plasma was stabilized for nearly 30 days in the presence of inhibitors compared with plasma spiked with BNP in the absence of inhibitors. In these studies, the following combination of inhibitors was used: AEBSF, i.e., [4-(2-aminoethyl)benzenesulfonylfluoride]), (200 µg/ml), antipain (100 µg/ml), benzamidine (14 mM) and PPACK (35 µg/ml).

### SUMMARY OF THE INVENTION

In one of its aspects, the present invention provides a method for stabilizing BNP in a body fluid sample, particularly, a blood based sample, such as plasma or serum, preferably a plasma sample. In accordance with the invention, stabilizing BNP reduces its degradation by endogenous proteases in the sample. The method of the invention comprises the addition of stabilizing components to blood samples that contain BNP, either exogenously added or endogenous BNP, or to vessels or containers that will receive blood samples containing BNP. The stabilizing components comprise one or more protease inhibitor compounds that reduce, protect against, or prevent proteolytic degradation of BNP. The inhibitor compounds include acetyl-leu-leu-arginal (leupeptin), N-(Nα-carbonyl-Arg-Val-Arg-al)Phe (antipain) and H-D-Phe-Phe-Arg-chloromethylketone (PPACK), as well as D-Phe-Pro-Arg-chloromethylketone (PPRACK) and diisopropylfluorophosphate (DFP). According to this invention, the compounds can be used alone or in combination. The combinations can comprise two, three, or more of the stabilizing components in admixture. In addition, the inhibitor 4-(2-aminoethyl)benzenesulfonylfluoride (AEBSF) can be used alone or in combination with the foregoing inhibitors. Examples of preferable BNP inhibitor combinations include leupeptin and PPACK; antipain and PPACK; leupeptin and PPRACK; antipain and PPRACK; leupeptin, antipain and PPACK; and leupeptin, antipain, PPRACK and DFP. Preferred inhibitors, especially for practical applications, are PPACK or PPRACK. The BNP inhibitors as described herein offer improved stabilization of BNP relative to previously used compounds.

In another aspect, the invention provides a stabilized composition of BNP comprising stabilizing inhibitor components, alone or in combination. More specifically, the composition can comprise components selected from PPACK alone; antipain alone, or leupeptin alone; a combination of leupeptin and PPACK; a combination of antipain and PPACK; a combination of leupeptin and antipain; a combination of leupeptin, antipain and PPACK; and a combination of leupeptin, antipain, PPACK and DFP, or analogs, variants, or derivatives thereof, or inhibitors which are structurally and/or functionally related thereto. For example, when PPACK is used, it will be understood throughout that PPRACK can also be used alone, or in the aforementioned combinations. Preferably, the stabilizing combination of components includes leupeptin and PPACK (or PPRACK). The stabilizing components as described herein can be added to, i.e., spiked into, a blood sample to stabilize the endogenous BNP in the sample, and/or to stabilize exogenous BNP that can also be spiked into a sample. The methods and compositions as described herein offer significant improvement of BNP stability in blood samples, including plasma and serum samples. (e.g., Example 5).

In another of its aspects, the present invention provides a procedure for stabilizing BNP-containing blood samples by directly adding the above-mentioned inhibitor components, and combinations thereof, to collected blood samples, preferably plasma samples, prior to, at the time of, or shortly after collection and before testing in a laboratory or clinical setting, or before storage. These components serve as BNP inhibitor additives to fresh samples, as well as to thawed samples, particularly, freshly thawed samples, of blood, plasma, or serum. Preferred is the use of PPACK in an aqueous composition to stabilize BNP in fresh or thawed plasma.

In yet another aspect, the present invention provides a blood collection container or vessel, such as a vacuum tube (e.g., Vacutainer^{™}) used in the collection of whole blood, comprising one, or a combination of, e.g., a cocktail of, the inhibitor components selected from, for example, antipain, leupeptin, PPACK, PPRACK and DFP. Blood samples, particularly, BNP-containing samples, are collected in such blood collection vessels or containers having therein one or more of the inhibitors as described. In this aspect, the pre-added inhibitor components, such as leupeptin and PPACK, are present to stabilize BNP in a collected blood or plasma sample immediately upon introduction of the sample into the container or vessel.

In another aspect, the present invention provides stable plasma controls for BNP determination. According to this aspect of the invention, controls can be made by spiking a fixed amount of exogenously added BNP, e.g., a recombinantly produced or synthetic form of BNP, such as BNP-32, (D.R. Murdoch et al., 1999, "Disparity between studies of the stability of BNP in blood: comparison of endogenous and exogenous peptide", *Heart,* 81:212), into a human blood-based matrix, e.g., a plasma matrix, wherein the matrix contains one or more of the added inhibitor components according to the present invention, e.g., leupeptin, antipain, PPACK, PPRACK, DFP, or combinations thereof. This feature of the invention overcomes prior difficulties in the preparation of plasma matrix controls due to the rapid degradation of spiked BNP by endogenous proteases during the preparation and value assignment procedure for such controls.

In yet another aspect, the present invention involves the introduction of at least one of, or a cocktail of a combination of, the inhibitor components antipain, leupeptin, PPACK, PPRACK, and/or DFP into BNP-containing blood samples, including plasma and serum samples. The invention provides stable compositions comprising BNP and the inhibitor components. As a preferred example, the inclusion of the combination of leupeptin and PPACK to blood-based matrices prior to the addition of exogenous, e.g., synthetic, BNP provides a stable control material preparation, for example, for commercial controls of BNP that are stable in a human or animal plasma matrix.

Further aspects, features and advantages of the present invention will be appreciated upon a reading of the detailed description of the invention.

### DETAILED DESCRIPTION OF THE INVENTION

The present invention relates to methods and compositions comprising one or more protease inhibitor components that inhibit or reduce the degradation of the cardiac peptide hormone BNP in blood samples, particularly plasma and serum samples. BNP degradation during sample collection, storage and testing can cause falsely decreased or negative test results, and erroneous sample classification, for clinically or medially tested patient samples. In accordance with the present invention, the addition of one or more of the newly determined BNP stabilizing components as described herein to blood, particularly blood plasma, significantly reduces or inhibits BNP degradation and allows more accurate and reliable sample interpretation. The methods and compositions involving the BNP stabilizing materials according to the present invention provide more accurate BNP concentration measurements in routine assays for the diagnosis and management of patients being tested for, or suspected to have, cardiac failure and/or cardiac disease.

The components which have been found, alone or in combination, e.g., as a cocktail of more than one component, to have superior BNP stabilizing properties according to this invention include the protease inhibitors acetyl-leu-leu-arginal (leupeptin); N-(Nα-carbonyl-Arg-Val-Arg-al)Phe (antipain), (Sigma Aldrich, St. Louis, MO; Calbiochem, San Diego, CA); and H-D-Phe-Phe-Arg-chloromethylketone (PPACK), (Bachem-Penninsula Labs, San Carlos, CA), as well as H-D-Phe-Pro-Arg-chloromethylketone (PPRACK), (Calbiochem, San Diego, CA) and diisopropylfluorophosphate (DFP), (Calbiochem, San Diego, CA). In addition, the inhibitor 4-(2-aminoethyl)benzenesulfonylfluoride (AEBSF), or compounds structurally related to AEBSF, can be used alone or in combination with the foregoing inhibitors. These stabilizing components, or compositions comprising these components, can be used to stabilize endogenous BNP in blood samples, as well as BNP that is exogenously added to, e.g., spiked into, blood samples.

In accordance with the present invention, one or more of the above inhibitors stabilizes BNP and reduces or prevents its degradation by proteases in a blood (plasma) sample. Alone, and in combination with antipain, leupeptin, or both, PPACK has been particularly newly found to vastly improve the stability of BNP. When added directly to blood plasma samples of animals (mammals), preferably humans, the inhibitors, preferably, leupeptin and PPACK, or antipain and PPACK, in combination, stabilized BNP in the samples at room temperature and at 4°C, even after repeated freezing and thawing. (Example 1).

The stabilizing capacity of the aforementioned inhibitors was determined based on an evaluation of the structure of BNP, which comprises regions that could be specifically sensitive to proteolysis, for example, a region of the circular portion of mature BNP-32 comprising the residues -Phe-Gly-Arg-. Accordingly, inhibitors of the present invention, e.g., PPACK and PPRACK without limitation, having structural similarity to the Phe-Gly-Arg residues comprising BNP, are especially suited to stabilizing BNP. Without wishing to be bound by theory, having a structural similarity to BNP provides an inhibitor with preferably better BNP-stabilizing function. In addition, in the control materials and stabilized compositions as further described herein, BNP can be modified so as to protect its intrinsic arginine (Arg) residues and related fragments from degradation, for example, by replacement of the natural amino acid residues involved in degradation of BNP-32 by molecules that can increase the resistance of BNP to proteolysis. As a nonlimiting example, a more stable BNP can contain D-Arg instead of native L-Arg. Further, more effective BNP stabilizing compounds can also be designed using this rationale.

The present invention also encompasses stabilizing methods or compositions which additionally comprise one or more PPACK analogs, variants, or derivatives as An example of a compound related to PPACK that is suitable for use according to the present invention is PPRACK (D-Phe-Pro-Arg-chloromethylketone), (Calbiochem, San Diego, CA).

In a particular related embodiment of the present invention, the inhibitors diisopropylfluorophosphate (DFP), (Calbiochem, San Diego, CA) and PPRACK have been shown to stabilize BNP as efficiently as antipain, leupeptin and PPACK. (See, Example 5, Table 11). Accordingly, the invention encompasses DFP and/or PPRACK used alone or in combination with one or more of antipain, leupeptin and PPACK in the compositions and methods described herein.

This invention embraces numerous practical applications, including, (i) adding the described BNP degradation inhibitors in the process of collecting blood or plasma samples to prevent sample BNP degradation during pre-test manipulations; (ii) adding the inhibitors to blood or plasma samples post-collection to prevent degradation of BNP during preparation for testing and during testing; and (iii) adding the inhibitors to plasma or a serum-free base for the preparation of controls or medical decision pools using either endogenous or synthetic BNP. These applications will be described further herein.

In one embodiment according to this invention, a method is provided in which one or more of the stabilizing components leupeptin, antipain, PPACK, PPRACK, and/or DFP are introduced into human or animal blood samples to stabilize the BNP therein. Preferably, the sample is a plasma sample. More preferably, the sample is a human plasma sample. The stabilization of BNP in blood samples of animals (mammals) other than humans, e.g., dogs, cats, cattle, horses, sheep or pigs, is also envisioned, such as for veterinary applications.

The BNP-stabilizing components of the present invention include the protease inhibitors leupeptin, antipain, PPACK, PPRACK, and/or DFP, which are used alone, or in combination, to stabilize BNP, i.e., reduce or eliminate its degradation, in a blood or plasma sample. Although each inhibitor component shows efficacy in stabilizing BNP in plasma samples over time, combinations of the inhibitor components, such as, for example, a combination of leupeptin and PPACK, or a combination of antipain and PPACK, or a combination of antipain, leupeptin and PPACK, yield significant BNP stabilizing effects over time at room temperature and 4°C, and upon thawing of frozen samples. (See, e.g., Examples 1-3 and 7). Thus, the methods and compositions of the present invention allow blood samples containing BNP to be accurately analyzed, with reliable values for BNP obtained, under conditions routinely encountered in medical practice, such as in clinics, clinical laboratories, hospital wards and physicians' offices. In addition, the present invention provides advantageous methods and compositions to allow for more efficient, facile blood collection and transport of blood samples to a clinic or laboratory for BNP analysis.

For use as a stabilizing component in blood and plasma samples, the protease inhibitors as described herein are present in the sample in a BNP stabilizing amount. For example, and without limitation, acetyl-leu-leu-arginal (leupeptin) is present in a sample in an amount of from about 0.5 µglml to about 55 µg/ml, preferably from about 5 µg/ml to about 50 µg/ml and more preferably from 45 µg/ml to about 55 µg/ml; H-D-Phe-Phe-Arg-chloromethylketone (PPACK) or D-Phe-Pro-Arg-chloromethylketone (PPRACK) is present in a sample in an amount of from about 0.35 µg/ml to about 38 µg/ml, preferably from about 3.5 µg/ml to about 35 µg/ml, and more preferably from about 32 µg/ml to about 38 µg/ml; and antipain is present in a sample in an amount of from about 0.5 µg/ml to about 55 µg/ml, preferably from about 5 µg/ml to about 50 µg/ml, and more preferably from about 45 µg/ml to about 55 µg/ml. DFP is preferably present in an amount of about 2 µg/ml to about 200 µg/ml; more preferably, in an amount of about 18 µg/ml (100 µM).

Another embodiment of the invention embraces a stabilized composition comprising the BNP stabilizing components described herein, alone or in combination, in a plasma matrix, preferably, a human plasma matrix. The stabilized composition, which is suitable for use as a control for clinical and medical use, comprises one or more of leupeptin, antipain and/or PPACK which reduce or prevent proteolytic degradation of BNP in the sample, i.e., the plasma matrix. In addition, the stabilized composition can comprise DFP and/or PPRACK alone, in combination with each other, or in combination with other inhibitors according to this invention. When combinations of the stabilizing components are used, the stabilized composition can comprise, as nonlimiting examples, leupeptin and PPACK in combination; leupeptin and antipain in combination; antipain and PPACK in combination; antipain, leupeptin and PPACK in combination; leupeptin and PPRACK in combination; antipain and PPRACK in combination; antipain, leupeptin and PPRACK in combination; DFP and PPACK in combination; DFP and PPRACK in combination; DFP and antipain in combination; DFP and leupeptin in combination; DFP, antipain, leupeptin and PPACK in combination; DFP, antipain, leupeptin and PPRACK in combination; DFP, antipain, leupeptin, PPACK and PPRACK in combination, so as to provide a cocktail of these stabilizing ingredients.

The stabilizing ingredients can be formulated or combined alone or together, lyophilized if desired, or dissolved in aqueous solution or buffer prior to use, and introduced as an additive to freshly prepared laboratory samples. Alternatively, the stabilizing ingredients comprising one or more of antipain, leupeptin, PPACK, PPRACK, or DFP, can be provided directly as an aqueous solution, a BNP stabilizing effective amount of which can be added to the plasma, or blood sample, as needed or desired, before the initial measurement of BNP. Preferably, yet without limitation, the inhibitor(s) according to the present invention are added within approximately 5 to 30 minutes or less after separation of plasma from whole blood via centrifugation collection; samples are preferably frozen for storage. If serum were to be used as the sample type, inhibitors are added to the collection tube prior to whole blood collection as the process of clotting destroys BNP.

The stabilizing components can be provided in concentrated form, such that a mere dropperful of a concentrated preparation of the protease inhibitors, for example, can be added to a blood or plasma sample without causing any significant dilution of the blood or plasma. Accordingly, the stabilizing components can be provided in a dropper vial or container, e.g., a container suitable for receiving a dropper or similar device, or even a syringe, for ease in dispensing the components into a sample. Preferably, the components are in combination, for example, a combination of leupeptin and PPACK, or a combination of antipain and PPACK, in the concentrated formulation to be added to a sample. In addition, a dropper vial or container housing a protease inhibitor composition of the invention can be included in a kit for performing an assay for BNP detection in blood or plasma samples. Such an aspect for providing stable BNP is particularly advantageous for commercial applications, since plasma is routinely separated at variable intervals prior to actual analytical measurement of BNP as an analyte in a patient's sample.

For guidance, concentrated inhibitor(s) can provided at a concentration of approximately 5-10 mg/ml in frozen form, as a nonlimiting example; or as a dry powder, at approximately 5-10 mg/vial as a nonlimiting example, so as to allow straightforward dilution to about 5-10 mg/ml. A 5-10 mg/ml solution(s) typically represents a 100x-200x stock material for the further addition to BNP-containing samples, e.g., at a ratio of 1 volume to 99-199 volumes.

In another embodiment, control materials, such as medical decision pools, encompass materials that are made in the same blood-based matrix, e.g., plasma, as the sample undergoing analysis, e.g., a human plasma sample. Such control materials must be accurate and contain stable components, as they serve as standards against which clinical decisions relating to patient treatment and outcome are made. Illustratively, the controls have set values or levels of included materials, such as BNP; the set values or levels correspond to a cut point, i.e., a value that is used to make a medical determination or decision involving a patient, for example, the amount or level of BNP present in a patient's sample. Controls having particular ranges of set levels of stabilized BNP based on particular cut points are able to be prepared for various uses, e.g., screening of a patient's sample for risk of heart attack, infarction, further cardiac disease and the like; monitoring a cardiac patient's therapy; and/or staging a cardiac patient as to degree or severity of cardiac disease.

In this embodiment of the present invention, the stabilizing components, e.g., leupeptin, antipain, PPACK, PPRACK, DFP and combinations thereof, are advantageous in producing such control materials involving BNP in a blood matrix to prevent or significantly reduce the degradation of the BNP over time, and/or after freezing and thawing. Use of one or more of the BNP-stabilizing inhibitors according to the present invention to prepare control materials allows large numbers of control samples to be made (e.g., on the order of thousands) using large quantities of material (e.g., plasma). In addition, these control materials can be stored and remain stable over time, since the levels of components such as BNP in these control materials remain stable over time. Further, the values of the BNP, such as spiked or exogenously added BNP, do not vary significantly from the initial set value or cut point of the control so that read-back values remain virtually constant and reliably stationary in the stored control materials. In accordance with this embodiment, the inhibitors, alone or in combination, can be added to the blood-based (e.g., plasma) matrices prior to, or at the time of, the addition of exogenous BNP (e.g., synthetic BNP), to prepare the control materials for use in BNP analysis and quantification of patient blood samples.

In another embodiment, this invention provides a composition of stabilizing components, namely, one or more of the inhibitors leupeptin, antipain, PPACK, PPRACK, DFP, or combinations thereof, present in a blood collecting tube or vacutainer tube for use at the time of collecting whole blood. With the stabilizing components added prior to the collection of blood, any BNP present in the sample is stabilized at the time of collection. The stabilizing composition of the invention can comprise the protease inhibitors as described, e.g., leupeptin, antipain, PPACK, PPRACK, and/or DFP alone or in combination in the collection tube, either in a small amount of concentrated aqueous solution, or in a lyophilized form which solubilizes upon addition of the blood sample, or which is solubilized with a small amount of aqueous solution just prior to the collection of the blood sample. In order to avoid dilution of the collected sample, it is recommended to use a volume of the concentrated inhibitor(s) solution that does not exceed 1% of the sample volume. For example, not more than about 60 µl of concentrated inhibitor(s), or inhibitor mixture, should be added to a typical 6 ml collection tube.

In a particular embodiment, a combination of leupeptin and PPACK is added to blood based matrices, e.g., human plasma, prior to the addition of exogenous synthetic BNP to prepare control materials, medical decision pools, and the like. Controls and medical decision pools can also be prepared using a combination of stabilizing components and endogenous BNP.

In another of its embodiments, the present invention encompasses a procedure for stabilizing BNP-containing blood samples by directly adding the inhibitor components, and combinations thereof, to collected blood samples, preferably plasma samples, prior to, at the time of, or shortly after collection and before testing in a laboratory or clinical setting. These components serve as BNP inhibitor additives to fresh samples, as well as to thawed samples, particularly, freshly thawed samples, of blood, plasma, or serum. Preferred is the use of a combination of leupeptin and PPACK, or leupeptin and PPRACK, in an aqueous composition to stabilize BNP in fresh or thawed plasma.

Some samples have been found to have different aggressiveness toward BNP such that BNP decays very rapidly. This could be due to unusually rapid BNP decomposition in certain sets of samples as a result of more active or stronger proteases present in one sample versus another. For example, as shown in Table 1, a sample from one patient exhibited aggressive proteolytic degradation of BNP (i.e., > 50% loss within 24 hours after storage at 4°C); however, with the addition of proteases according to the present invention, such proteolysis was significantly curtailed.

**Table 1**

| Sample #371529* | 1st test-1 hour after being thawed | 2nd test after 4 hours at 4°C | 3rd test after 24 hours at 4°C | Recovery vs 1st test |
|---|---|---|---|---|
| | pg/ml BNP | pg/ml BNP | pg/ml BNP | % |
| Sample w/o inhibitor | 448.53 | 392.31 | 212.48 | 47.4 |
| | | | | |
| Sample with leupeptin (50 µg /ml) and PPACK (35 µl/ml) | 497.94 | 451.20 | 505.84 | 101.6 |

| | | | | |
|---|---|---|---|---|
| *: This sample represents a human Congestive Heart Failure (CHF) sample, New York Heart Association (NYHA) class III from ProMedDx (Norton, MA). | | | | |

Thus, the addition of inhibitor(s) to a sample is preferably done as soon as possible (e.g., within minutes; preferably within about 5 minutes or less) after collection. The use of the inhibitors according to the present invention affords good (e.g., ~90 %) recovery of exogenous BNP in human plasma stored at room temperature (~20°C) for approximately 44 hours. In addition, the following Table 2 shows a dose recovery of synthetic BNP (2000 pg/ml) spiked into human plasma samples (Intergen), with and without leupeptin (50 µg/ml and PPACK (35 µg/ml) as added inhibitors.

**Table 2**

| Sample | Dose Time 0 (Fresh) | Dose 24 hours at 4°C | Dose 90 hours at 4°C | % Recovery 24 hours | % Recovery 90 hours |
|---|---|---|---|---|---|
| Human Plasma w/o inhibitor | 1894.8 | 655.7 | 56.65 | 34.6 | 3.0 |
| Human plasma with inhibitors | 2311.4 | 2333.9 | 2360.3 | 101 | 102 |

However, for samples with higher BNP decay rates, an acceptable storage time may be much shorter. Thus, sample (and BNP) storage is preferably for a time that minimizes exposure of BNP to temperatures greater than 0°C, even in the presence of inhibitor(s).

In another aspect, the present invention provides stable plasma controls for BNP determination. According to this aspect of the invention, controls can be made-by-spiking a fixed amount of exogenously added BNP, e.g., a recombinantly produced or synthetic form of BNP, such as BNP-32, (D.R. Murdoch et al., 1999, "Disparity between studies of the stability of BNP in blood: comparison of endogenous and exogenous peptide", *Heart*, 81:212), into a human blood-based matrix, e.g., a plasma matrix, wherein the matrix contains one or more of the added inhibitor components according to the present invention, i.e., leupeptin, antipain and PPACK, or combinations thereof. This feature of the invention overcomes prior difficulties in the preparation of plasma matrix controls due to the rapid degradation of spiked BNP by endogenous proteases during the preparation and value assignment procedure for such controls. With one or more of the stabilizing and inhibitor components, e.g., leupeptin and PPACK (or PPRACK), in the plasma matrix sample, the sample has demonstrable stability, e.g., at 4°C for at least 17 hours, without loss of activity.

For example, Table 3 below presents the stability results of synthetic BNP-32 added to normal human plasma containing both leupeptin (50 µg/ml) and PPACK (35 µg/ml) in accordance with this invention:

**Table 3**

| Sample | Before storage | After storage | % change |
|---|---|---|---|
| | | (17 hours at 4°C) | |
| | BNP, pg/ml | BNP, pg/ml | |
| control plasma A | 45.03 | 45.1 | 0.2 |
| control plasma B | 419.69 | 416.2 | -0.8 |

Table 4 shows that at room temperature (RT), (22°C), the stability of BNP-32 in the plasma sample containing the inhibitor combination as described above with respect to Table 3 was only slightly inferior to its stability at 4°C.

**Table 4**

| | Before storage | 20 hours at 4°C | % Recovery vs 1st run (Time 0) | 20 hours at RT | % Recovery vs 1st run (Time 0) |
|---|---|---|---|---|---|
| Sample | | | | | |
| | BNP, pg/ml | | | BNP, pg/ml | |
| control plasma A | 45.1 | 45.9 | 101.7 | 43.9 | 97.3 |
| control plasma B | 391.7 | 453.2 | 115.7 | 424.4 | 108.3 |

### EXAMPLES

The following examples describe specific aspects of the invention to illustrate the invention and provide a description of the present methods for those of skill in the art.

### EXAMPLE 1

Experiments were conducted to test the efficacy of the BNP-stabilizing inhibitor compounds as described herein, both alone and in combination, utilizing an immunoassay for synthetic BNP-32 detection in samples.

The experiments described in this Example tested the stability of synthetic BNP-32 in plasma. To perform the experiments, protease inhibitors were added to ("spiked into") normal human plasma, followed by the addition of a known amount of BNP. Normal human plasma without added inhibitors was used as a control. The signal generated by BNP present in plasma samples with and without inhibitors was measured immediately after spiking and after storage of the samples at 4°C.

To generate an analytical signal to quantify the BNP concentration in the sample, two monoclonal BNP-specific antibodies (Shionogi & Co., LTD, Osaka, Japan; EP542255A1/B1; JP 3297392) were used. The first antibody was labeled with acridinium ester (AE) and the second antibody was labeled with biotin. The two antibodies bind to BNP molecules present in the plasma samples to form a complex; the formed complex was captured by paramagnetic particles (PMP) coated with streptavidin. PMP was separated by magnetic field and washed to remove non-specifically bound material. BNP-AE complexes retained on the PMP were treated with alkaline solution to produce a chemiluminescent signal, which was proportional to the amount of BNP captured; the signal was measured in relative light units or "RLU". The assay was carried out on an automated chemiluminescent system ADIVA:Centaur^{™} (Bayer Corporation, Tarrytown, NY) using BNP detection reagents, such as have been described. (e.g., B. Bluestein et al., 2002, "Development of an automated test for BNP as an aid in the diagnosis and evaluation of CHF on the Bayer ADVIA Centaur ACS", *Clinical Chemistry,* 48(S6):A85, Abstract C37). The results of the experiments are presented in Table 5.

In Table 5, control standards (S1-S7) were frozen in phosphate buffer until use. These standards were prepared gravimetrically by spiking synthetic BNP into physiological buffer solution. Column 1 of Table 5 indicates the samples tested: Standards, S1-S7; Intergen's Human Plasma Pool (IHPP), commercially available from Intergen (Milford, MA), comprising normal EDTA plasma collected from healthy human donors and tested negative for hepatitis B virus, hepatitis C virus and HIV by FDA approved methods; AEBSF (i.e., [4-(2-aminoethyl)benzenesulfonylfluoride]) inhibitor; antipain inhibitor; benzamidine inhibitor; PPACK inhibitor; and KCBB (Kansas City Blood Bank) Plasma Pool, which comprised pooled normal EDTA plasma from healthy human donors and tested negative for hepatitis B virus, hepatitis C virus and HIV by FDA approved methods.

Column 2 of Table 5 indicates the BNP concentration, [BNP], if present. Columns 3-8 of Table 5 show a stability profile of BNP in plasma samples and indicate times of assaying a sample for BNP. "RLU" indicates relative light units or photon counts as quantified in the assay. Column 9 of Table 5 indicates the percent signal recovered.

**Table 5**

| **Sample** | **Expected [BNP]** | **Day 0 (RLU)** | **72 hrs (RLU)** | **1 week (RLU)** | **≡2 weeks (RLU)** | **22 days (RLU)** | **4 weeks (RLU)** | **>4 weeks % Signal Recovered** |
|---|---|---|---|---|---|---|---|---|
| S1 | 0 pg/ml | 2205 | 2029 | 2062 | 1649 | 1882 | 2059 | 93.4 |
| S2 | 10 pg/ml | 4009 | 3742 | 3973 | 3528 | 4086 | 3917 | 97.7 |
| S3 | 20 pg/ml | 4816 | 5271 | 5136 | 4688 | 5292 | 5213 | 108.2 |
| S4 | 100 pg/ml | 17436 | 18260 | 17643 | 18495 | 20279 | 19088 | 109.5 |
| S5 | 500 pg/ml | 73578 | 95845 | 96215 | 93669 | 104537 | 101422 | 137.8 |
| S6 | 1000 pg/ml | 201952 | 199347 | 202699 | 212546 | 233157 | 215283 | 106.6 |
| S7 | 2000 pg/ml | 441342 | 454206 | 475178 | 487294 | 521597 | 499076 | 113.1 |
| Intergen's Human Plasma Pool, (IHPP), (No inhibitor) | 0 | 2030 | 1835 | 1666 | 1382 | 1586 | 1610 | 79.3 |
| IHPP, (No inhibitor) | 10ng/ml | 4071756 | 1963668 | 219239 | 5136 | 4435 | 3430 | 0.1 |
| IHPP + AEBSF (200 µg/ml out of a 100 mg/ml stock) | 10ng/ml | 4079471 | 3249767 | 2504809 | 4241 | 3812 | 2434 | 0.1 |
| IHPP + antipain (100 µg/ml) out of a 20 mg/ml stock) | 10ng/ml | 3985365 | 3600518 | 3379524 | 2400716 | 2085558 | 1469626 | 36.9 |
| IHPP+ benzamidine (14 mM out of a 1.43M stock) | 10ng/ml | 3927159 | 3342787 | 2568730 | 238972 | 108308 | 30024 | 0.8 |
| IHPP + PPACK (50 µg/ml out of a 5 mg/ml stock) | 10ng/ml | 4207901 | 4040859 | 3647828 | 2909220 | 781369 | 10684 | 0.3 |
| IHPP + AEBSF + antipain+ benzamidine + PPACK | 10ng/ml | 4083615 | 3929683 | 3845404 | 3682508 | 3610611 | 3167044 | 77.6 |
| KCBB Plasma Pool (no inhibitor) | 0 | 1651 | 1672 | 1703 | 1508 | 1639 | 1486 | 90.0 |
| KCBB Plasma Pool | 10ng/ml | 2211455 | 4597 | 3163 | 2330 | 2427 | 2018 | 0.1 |
| KCBB Plasma Pool + (AEBSF + antipain + benzamidine + PPACK) | 10ng/ml | 3847429 | 3748585 | 3548759 | 2679369 | 2640700 | 1658338 | 43.1 |

The above Table 5 presents data comparing two plasma samples, plasma from Intergen and plasma from KCBB. Plasma samples were spiked with an extremely high dose of synthetic BNP in order to facilitate monitoring of the BNP concentration. Plasma with BNP, but without added inhibitors, served as a negative control. BNP in KCBB samples without inhibitors was practically undetectable after 72 hours of storage; however, the same plasma with inhibitors demonstrated only a gradual decrease of BNP concentration, i.e., to ~92% of its initial value after the first week of storage. The potency of individual inhibitors was tested only for IHPP. PPACK appeared to be the most potent of the tested inhibitors and provided a sufficient degree of protection to exogenous BNP for at least three days, i.e., -95.9 % of BNP was recovered. Antipain also demonstrated good protective ability (-90 % recovery after 3 day storage). In this set of experiments, the activity of antipain outlasted that of PPACK. The effect of antipain could be observed for 4 weeks, while the activity of PPACK was essentially absent. However, as will be appreciated by the skilled practitioner, it is not uncommon for inhibitors to be unstable in some blood samples; therefore, the inhibitory activity of typically good inhibitors, e.g., PPACK, can deteriorate over time in some plasma samples, but not in all samples.

The above findings, along with the dynamics of blood sample differences, provides that an antipain and PPACK combination is quite suitable in the present invention, and may be desired in some cases instead of a single inhibitor. But for practical purposes, either PPACK or antipain alone would also provide acceptable BNP stability. Because antipain generally exhibits an activity spectrum similar to that of leupeptin, leupeptin was included in further tests. (See Table 7, Example 2).

Table 6 below presents data showing that PPACK alone successfully stabilized BNP in a sample to a level that is acceptable to reduce or inhibit BNP degradation. Thus, in accordance with the present invention, the inhibitory activity of a combination of inhibitors, with PPACK included in the mixture, allows for longevity of the inhibitory activity so as to stabilize BNP.

**Table 6**

| Storage Condition | | | | | | |
|---|---|---|---|---|---|---|
| | Frozen without storage at 4°C | 1 hour at 4°C before freezing | 3 hours at 4°C before freezing | 24 hours at 4°C before freezing | 48 hours at 4°C before freezing | % recovery versus 1st run |
| Samples | | | | | | |
| | BNP, pg/ml | BNP, pg/ml | BNP, pg/ml | BNP, pg/ml | BNP, pg/ml | |
| Human Plasma (HP) w/o BNP (negative control) | 10.6 | | | | | |
| Samples with spiked synthetic BNP: | | | | | | |
| HP w/o inhibitor | 782.6 | 678.3 | 652.6 | 455.2 | 288.3 | 36.8 |
| HP + PPACK, 0.35 µg/ml | 806.7 | 728.2 | 772.6 | 732.9 | 745.3 | 92.4 |
| HP + PPACK, 3.5 µg/ml | 744.9 | 792.4 | 801.4 | 784.5 | 809.9 | 108.7 |
| HP + PPACK, 35 µg/ml | 822.5 | 778.0 | 824.4 | 797.3 | 840.8 | 102.2 |

### EXAMPLE 2

This Example presents data from additional assays performed to evaluate the effect of the inhibitor components PPACK, leupeptin, and antipain, alone and in combination, on the stability of synthetic BNP-32 in plasma over time. Plasma samples were spiked with synthetic BNP-32 and stored at 4°C for varying periods of time, i.e., Day 0, 24 hours and ≅90 hours, prior to testing. The assay to detect and quantify the presence of BNP at a particular time point was the same as that described in Example 1. Intergen human plasma (IHP) samples containing EDTA (K₂ EDTA or K₃ EDTA, in an amount of about 1.8 g/l of blood, or equivalent to -10.8 mg per 6 ml collection tube), (Intergen #01D1707, Intergen, Milford, MA) were used. The standards were as described in Example 1. The results of the experiments performed in this Example are presented in Table 7.

**Table 7**

| **Sample** | **[BNP]** | **Amount of BNP Present Before Storage, Day 0** | **Amount of BNP Present After 24 hr/4°C** | **Amount of BNP Present After 90 hr/4°C** | **BNP Amount % Change** |
|---|---|---|---|---|---|
| S1 | 0 pg/ml | 7.21 | | 1.11 | NA |
| S2 | 10 pg/ml | 12.38 | 18.93 | 15.76 | 27.4 |
| S3 | 20 pg/ml | 83.56 | 72.59 | 73.76 | -11.7 |
| S4 | 100 pg/ml | 152.58 | 142.33 | 152.99 | 0.3 |
| S5 | 500 pg/ml | 696.35 | 707.44 | 710.59 | 2.0 |
| S6 | 1000 pg/ml | 1480.85 | 1501.80 | 1524.01 | 2.9 |
| S7 | 2000 pg/ml | 3161.01 | 3266.73 | 3081.56 | -2.5 |
| IHP. no inhibitor | 0 | 0 | 1.42 | 1.69 | NA |
| IHP, no inhibitor | 1000 µg/ml | 1894.84 | 655.72 | 56.65 | -97.0 |
| IHP, + antipain, (50 µg/ml) | 1000 µg/ml | 2194.06 | 2234.34 | 1880.94 | -14.3 |
| IHP, + leupeptin, (50 µg/ml) | 1000 pg/ml | 2290.08 | 2503.78 | 2133.75 | -6.8 |
| IHP, + PPACK, (50 µg/ml) | 1000 pg/ml | 2243.59 | 2444.60 | 2308.43 | 2.9 |
| IHP, + antipain, (50 µg/ml) + leupeptin, (50 µg/ml) | 1000 pg/ml | 2125.09 | 2248.56 | 2043.75 | -3.8 |
| IHP, + antipain, (50 µg/ml) + PPACK, (50 µg/ml) | 1000 pg/ml | 2139.31 | 2237.43 | 2180.14 | 1.9 |
| IHP, + leupeptin, (50 µg/ml) + PPACK, (50 µg/ml) | 1000 pg/ml | 2311.37 | 2333.90 | 2360.25 | 2.1 |
| IHP, + antipain, (50 µg/ml) + leupeptin, (50 µg/ml) + PPACK, (50 µg/ml) | 1000 pg/ml | 2171.76 | 2412.27 | 2276.82 | 4.8 |

As can be seen from the results presented in Table 7, the inclusion of the stabilizing components, leupeptin, antipain, or PPACK (50 µg/ml) alone or together, greatly improved the stability of BNP following refrigerated storage of plasma samples at 4°C for differing amounts of time, followed by testing of the samples. In particular, the last column in Table 7 shows the overall changes in BNP concentration during the stability study. A negative number, for example, -14.3 % in the case of antipain, indicates that the loss of BNP resulted in 14.3 % of the initial amount of BNP. Although the results support the use of PPACK alone to stabilize BNP in samples, BNP stability can be enhanced according to this invention by combining one or more of the inhibitors as described herein, especially for long-term stability as is represented in Table 7 above.

### EXAMPLE 3

The experiments conducted in this Example evaluated the stability of endogenous BNP in plasma samples (i.e., human CHF (NYHA class III) plasma). Plasma samples containing endogenous BNP were purchased from ProMedDx (Norton, MA) and were stored frozen. On the day of assay, the samples were thawed prior to use. Thawed samples were utilized in about 1 hour following thawing. Inhibitors were added immediately after the sample was thawed. The results are presented in Table 8.

In Table 8, Column 2 reflects amount of BNP present in a plasma sample thawed and tested on Day 0. Column 3 of Table 8 presents the amount of BNP present in the Day 0 plasma sample assayed after about 4 hours of storage at 4°C. Column 4 of Table 8 presents the amount of BNP present in the same Day 0 plasma sample assayed after about 24 hours of storage at 4°C. The assay was performed as described in the above Examples.

**Table 8**

| **Sample** | **Day 0 BNP stability (pg/ml)** | **Storage for ≅4 hr, 4°C BNP stability (pg/ml)** | **Storage for ≅24 hr, 4°C BNP stability (pg/ml)** |
|---|---|---|---|
| S1 | 1.34 | 0.04 | 0.94 |
| S2 | 14.65 | 16.13 | 14.74 |
| S3 | 75.91 | 81.50 | 77.89 |
| S4 | 151.92 | 139.39 | 149.89 |
| S5 | 732.80 | 689.96 | 725.19 |
| S6 | 1494.21 | 1555.13 | 1451.88 |
| S7 | 3041.30 | 3180.49 | 3159.16 |
| Plasma Sample (NYHA Class III), no inhibitor | 448.53 | 392.31 | 212.48 |
| Plasma Sample + leupeptin (50 µg/ml) + PPACK (50 µg/ml) | 497.94 | 451.20 | 505.84 |
| Blank Plasma (negative control -no BNP) | 0.71 | 1.83 | 1.84 |

The results from the above Examples confirm that improved stability is conferred upon blood plasma samples in the presence of the BNP stabilizing/inhibitor components according to the present invention.

### EXAMPLE 4

Experiments were performed to assess BNP stabilization in animal serum and human serum versus human plasma. Tables 9 and 10 below illustrate the efficacy of PPACK and leupeptin as BNP stabilizers in three different media, namely, bovine fetal serum (Biocell Labs, Rancho Dominguez, CA), normal human serum (Biocell Labs), and normal human plasma (Intergen). The results indicate that both human and animal sera behave much more aggressively than plasma with respect to BNP degradation; even at the highest tested concentration of inhibitors, the decay of peptide was rapid. Notwithstanding, in all conditions, the addition of PPACK and leupeptin was found to significantly inhibit BNP degradation:

**Table 9**

| Storage Conditions | | | | | |
|---|---|---|---|---|---|
| | Frozen w/o storage at 4°C | 3 hours at 4°C before freezing | 24 hours at 4°C before freezing | 48 hours at 4°C before freezing | Recovery vs 1st run (Time 0) |
| Samples with spiked synthetic BNP | BNP, pg/ml | BNP, pg/ml | BNP, pg/ml | BNP, pg/ml | % |
| Bovine Fetal Serum (BFS) w/o inhibitor | 726.8 | 523.5 | 331.8 | 215.1 | 29.6 |
| BFS + 0.35 µg PPACK/ml | 789.2 | 680.4 | 624.8 | 515.3 | 65.3 |
| BFS + 3.5 µg PPACK/ml | 783.8 | 727.7 | 671.6 | 601.2 | 76.7 |
| BFS + 35 µg PPACK/ml | 769.8 | 798.8 | 729.1 | 651.3 | 84.6 |
| | | | | | |
| Human Serum (HS) w/o inhibitor | 117.4 | 21.9 | 0.0 | 0.0 | 0.0 |
| HS + 0.35 µg PPACK/ml | 338.2 | 228.3 | 149.0 | 87.5 | 25.9 |
| HS + 3.5 µg PPACK/ml | 305.3 | 227.0 | 160.4 | 97.0 | 31.8 |
| HS + 35 µg PPACK/ml | 305.3 | 249.5 | 178.9 | 115.0 | 37.7 |
| | | | | | |
| Human Plasma (HP) w/o inhibitor | 782.6 | 652.6 | 455.2 | 288.3 | 36.8 |
| HP + 0.35 µg PPACK/ml | 806.7 | 772.6 | 732.9 | 745.3 | 92.4 |
| HP + 3.5 µg PPACK/ml | 744.9 | 801.4 | 784.5 | 809.9 | 108.7 |
| HP + 35 µg PPACK/ml | 822.5 | 824.4 | 797.3 | 840.8 | 102.2 |

**Table 10**

| Storage Conditions | | | | | |
|---|---|---|---|---|---|
| | Frozen w/o storage at 4°C | 3 hours at 4°C before freezing | 24 hours at 4°C before freezing | 48 hours at 4°C before freezing | Recovery versus 1st run |
| | | | | | |

| Samples with spiked synthetic BNP | BNP, pg/ml | BNP, pg/ml | BNP, pg/ml | BNP, pg/ml | % |
|---|---|---|---|---|---|
| | | | | | |
| Bovine Fetal Serum (BFS) w/o inhibitor | 726.8 | 523.5 | 331.8 | 215.1 | 29.6 |
| BFS + leupeptin, 0.5 µg/ml | 712.9 | 639.6 | 537.2 | 424.9 | 59.6 |
| BFS + leupeptin, 5 µg/ml | 845.0 | 672.2 | 601.7 | 498.9 | 59.0 |
| BFS + leupeptin, 50 µg/ml | 810.5 | 751.1 | 707.0 | 614.5 | 75.8 |
| | | | | | |
| Human Serum (HS) w/o inhibitor | 117.4 | 21.9 | 0.0 | 0.0 | 0.0 |
| HS + leupeptin, 0.5 µg/ml | 227.7 | 129.0 | 45.1 | 11.4 | 5.0 |
| HS + leupeptin, 5 µg/ml | 311.1 | 203.0 | 135.6 | 74.3 | 23.9 |
| HS + leupeptin, 50 µg/ml | 297.9 | 274.2 | 217.5 | 156.6 | 52.6 |
| | | | | | |
| Human Plasma (HP) w/o inhibitor | 782.6 | 652.6 | 455.2 | 288.3 | 36.8 |
| HP + leupeptin, 0.5 µg/ml | 765.2 | 751.3 | 696.1 | 672.6 | 87.9 |
| HP + leupeptin, 5 µg/ml | 679.0 | 828.3 | 807.5 | 784.4 | 115.5 |
| HP + leupeptin, 50 µg/ml | 867.2 | 795.6 | 763.0 | 798.3 | 92.1 |

### EXAMPLE 5

Example 5 presents a comparative evaluation of several different inhibitors. Table 11 below compares inhibitors of serine proteases associated with thrombolytic activity. The inhibitors were applied at their usual functional concentrations. All inhibitors except for PPACK were purchased from Calbiochem (San Diego, CA). Inhibitors such as aprotinin and benzamidine are known and were used for comparative purposes with the novel inhibitor compounds described herein. AEBSF ([4-(2-aminoethyl)benzenesulfonylfluoride)]) represents an inhibitor related to phenylmethylsulfonylfluoride (PMSF). The data indicate the low efficacy of aprotinin and the intermediate efficacy of AEBSF and benzamidine in stabilizing BNP. However, as newly provided herein, DFP (diisopropylfluorophosphate) and PPRACK (D-Phe-Pro-Arg-chloromethylketone) were found to act as efficiently as antipain, leupeptin and PPACK for BNP stabilization.

**Table 11**

| Storage Condition | | | | | | |
|---|---|---|---|---|---|---|
| | 1st run (Time 0) | 3 hours at 4°C | 17 hours at 4°C | 40 hours at 4°C | 6 days at 4°C | |
| | BNP, pg/ml | BNP, pg/ml | BNP, pg/ml | BNP, pg/ml | BNP, pg/ml | % Recovery vs 1st run (Time 0) |
| Plasma w/o inhibitor | 815.0 | 766.4 | 322.5 | 89.8 | 2.4 | 0.3 |
| Plasma with inhibitor tested: | | | | | | |
| Aprotinin, 100 µg/ml (-700 kallikrein inhibitory units/ml) | 792.4 | 849.7 | 680.0 | 490.9 | 167.4 | 21.1 |
| Benzamidine, 15 mM | 830.6 | 806.8 | 860.5 | 813.8 | 686.3 | 82.6 |
| AEBSF, 0.5 mg/ml (-2 mM) | 741.7 | 768.6 | 715.2 | 735.7 | 631.9 | 85.2 |
| DFP, ~30 mM | 853.3 | 809.6 | 859.8 | 882.3 | 830.8 | 97.4 |
| PPRACK, 50 µg/ml | 892.0 | 894.6 | 957.6 | 939.7 | 896.2 | 100.5 |
| antipain 50 µg/ml | 828.3 | 894.2 | 932.4 | 944.2 | 822.2 | 99.3 |
| leupeptin, 50 µg/ml | 792.0 | 896.9 | 864.4 | 873.7 | 835.9 | 105.5 |
| PPACK 35 µg/ml | 926.4 | 973.9 | 928.5 | 930.5 | 920.3 | 99.3 |

### EXAMPLE 6

The experiments shown in this example present the results of the titration of PPACK (Table 12) and antipain and leupeptin (Table 13) in different lots of human plasma. Data were collected following PPACK, antipain, or leupeptin titration in samples of normal human plasma (Intergen) spiked with synthetic BNP. For PPACK, three normal human plasma samples (A, B and C) were used. The results for the PPACK titration support an approximately 90% or greater recovery of BNP stored in plasma with PPACK at ~3.9 µg/ml and higher. (Table 12). The results for the antipain and leupeptin titration indicate that these inhibitors provide good protection for BNP stability in plasma at ~50 µg inhibitor/ml.

**Table 12**

| Storage Condition | | | | | | |
|---|---|---|---|---|---|---|
| | 1st run (Time 0) | 3 hours at 4°C | 17 hours at 4°C | 40 hours at 4°C | 6 days at 4°C | Recovery versus 1st run (Time 0) (%) |
| | BNP, pg/ml | BNP, pg/ml | BNP, pg/ml | BNP, pg/ml | BNP, pg/ml | |
| Plasma w/o inhibitor | | | | | | |
| Plasma A | 815.0 | 766.4 | 322.5 | 89.8 | 2.4 | 0.3 |
| Plasma B | 754.5 | 696.2 | 504.6 | 295.5 | 4.3 | 0.6 |
| Plasma C | 671.9 | 705.0 | 242.5 | 14.6 | 2.1 | 0.3 |
| | | | | | | |
| PPACK in A, 0.43 µg/ml | 875.5 | 886.1 | 721.3 | 822.2 | 701.7 | 80.1 |
| PPACK in A, 1.3 µg/ml | 872.1 | 897.2 | 840.2 | 882.5 | 821.9 | 94.2 |
| PPACK in A, 3.89 µg/ml | 829.2 | 896.7 | 854.5 | 873.0 | 814.7 | 98.2 |
| PPACK in A. 11.7 µg/ml | 917.2 | 900.3 | 874.9 | 961.5 | 782.4 | 85.3 |
| PPACK in A, 35 µg/ml | 926.4 | 973.9 | 928.5 | 930.5 | 920.3 | 99.3 |
| | | | | | | |
| PPACK in B. 0.43 µq/ml | 853.5 | 806.0 | 794.4 | 815.6 | 711.2 | 83.3 |
| PPACK in B, 1.3 µg/ml | 913.5 | 868.3 | 822.7 | 800.8 | 761.7 | 83.4 |
| PPACK in B, 3.89 µg/ml | 817.7 | 831.3 | 800.3 | 856.8 | 732.1 | 89.5 |
| PPACK in B, 11.7 µg/ml | 831.8 | 927.9 | 868.6 | 913.1 | 787.9 | 94.7 |
| PPACK in B, 35 µg/ml | 987.7 | 982.7 | 863.1 | 897.5 | 849.5 | 86.0 |
| | | | | | | |
| PPACK in C, 1.3 µg/ml | 878.9 | 918.0 | 838.0 | 768.1 | 681.9 | 77.6 |
| PPACK in C, 3.89 µg/ml | 864.4 | 869.4 | 815.2 | 817.0 | 837.1 | 96.8 |
| PPACK in C, 11.7µg/ml | 918.5 | 912.5 | 843.5 | 838.8 | 868.2 | 94.5 |
| PPACK in C, 35 µg/ml | 923.7 | 897.0 | 849.3 | 913.8 | 921.1 | 99.7 |

**Table 13**

| Storage Condition | | | | | | |
|---|---|---|---|---|---|---|
| | 1st run (Time 0) | 3 hours at 4°C | 17 hours at 4°C | 40 hours at 4°C | 6 days at 4°C | Recovery versus 1st run |
| | BNP, pg/ml | BNP, pg/ml | BNP, pg/ml | BNP, pg/ml | BNP, pg/ml | (%) |
| Plasma w/o inhibitor | | | | | | |
| Plasma A | 815.0 | 766.4 | 322.5 | 89.8 | 2.4 | 0.3 |
| | | | | | | |
| Antipain in A, 0.62 µg/ml | 912.4 | 830.8 | 715.7 | 688.7 | 354.1 | 38.8 |
| Antipain in A, 1.85 µg/ml | 901.3 | 861.6 | 874.2 | 799.7 | 544.8 | 60.4 |
| Antipain in A, 5.56 µg/ml | 921.7 | 909.0 | 984.8 | 371.6 | 716.5 | 77.7 |
| Antipain in A, 16.7 µg/ml | 967.6 | 980.1 | 1033.1 | 932.4 | 854.4 | 88.3 |
| Antipain in A, 50 µg/ml | 828.3 | 894.2 | 932.4 | 944.2 | 822.2 | 99.3 |
| | | | | | | |
| Leupeptin in A, 0.62 µg/ml | 847.4 | 863.3 | 776.1 | 721.6 | 373.6 | 44.1 |
| Leupeptin in A, 1.85 µg/ml | 841.7 | 873.5 | 859.4 | 795.5 | 604.7 | 71.8 |
| Leupeptin in A, 5.56 µg/ml | 879.3 | 900.4 | 847.2 | 808.0 | 722.8 | 82.2 |
| Leupeptin in A, 16.7 µg/ml | 886.7 | 849.8 | 904.5 | 903.1 | 808.2 | 91.1 |
| Leupeptin in A, 50 µg/ml | 792.0 | 896.9 | 864.4 | 873.7 | 835.9 | 105.5 |

### EXAMPLE 7

Example 7 presents the results of various experiments to determine the effect of PPACK on the stability of endogenous BNP. To determine the stability of different patient samples under various times of refrigerated storage, 5 patient samples were kept under refrigeration for 0, 1, 8, 24, 48 and 144 hours. A companion set of samples was spiked with PPACK to a final concentration of 35 µg/mL to assess the ability of this inhibitor to reduce degradation of BNP. As shown in Table 14 below, PPACK markedly retarded degradation, even at time 0. The results were calculated as absolute recovery against the time 0 (1st run) measurement.

**Table 14**

| Storage Conditions | | | | | | | | | | | |
|---|---|---|---|---|---|---|---|---|---|---|---|
| Sample | 1 st run (Time 0) BNP, pg/ml | 1 hour at 4°C BNP, pg/ml | % recovery vs 1st run | 8 hours at 4°C BNP, pg/ml | % recovery vs 1st run | 24 hours at 4°C BNP, pg/ml | % recovery vs 1 st run | 48 hours at 4°C BNP, pg/ml | % recovery vs 1 st run | 144 hours at 4°C BNP, pg/ml | % recovery vs 1st run |
| 1 | 65.4 | 67.9 | 103.8 | 62.3 | 95.3 | 51.2 | 78.3 | 41.5 | 63.5 | 0.8 | 1.3 |
| 1p* | 88.9 | 92.1 | 103.6 | 83.4 | 93.9 | 79.0 | 88.9 | 89.5 | 100.7 | 86.9 | 97.8 |
| | | | | | | | | | | | |
| 2 | 185.5 | 174.4 | 94.0 | 155.4 | 83.8 | 123.3 | 66.5 | 108.1 | 58.3 | 10.1 | 5.4 |
| 2p | 254.0 | 240.3 | 94.6 | 249.1 | 98.1 | 236.3 | 93.1 | 251.2 | 98.9 | 242.7 | 95.6 |
| | | | | | | | | | | | |
| 3 | 124.0 | 113.7 | 91.7 | 100.2 | 80.9 | 85.6 | 69.1 | 74.6 | 60.2 | 23.3 | 18.8 |
| 3p | 159.4 | 169.8 | 106.5 | 156.2 | 98.0 | 157.7 | 98.9 | 164.5 | 103.2 | 157.5 | 98.8 |
| | | | | | | | | | | | |
| 4 | 171.3 | 158.3 | 92.4 | 123.1 | 71.8 | 121.7 | 71.1 | 106.8 | 62.3 | 7.1 | 4.2 |
| 4p | 197.8 | 193.8 | 98.0 | 188.6 | 95.3 | 189.4 | 95.7 | 187.1 | 94.6 | 198.3 | 100.3 |
| | | | | | | | | | | | |
| 5 | 109.6 | 113.0 | 103.1 | 97.3 | 88.8 | 84.9 | 77.5 | 65.2 | 59.5 | 5.7 | 5.2 |
| 5p | 151.7 | 149.9 | 98.8 | 147.5 | 97.2 | 141.0 | 92.9 | 151.8 | 100.0 | 156.8 | 103.3 |

| | | | | | | | | | | | |
|---|---|---|---|---|---|---|---|---|---|---|---|
| *: The number (e.g., 1) is the patient sample number and "1p" indicates patient 1 sample with added PPACK inhibitor. This notation follows for the rest of the patient samples 2-5. | | | | | | | | | | | |

The results presented in Table 14 show that at 24 hours, the average loss of BNP was 28% in samples with no inhibitor compared with an average loss of only 6.2% with PPACK added. By 48 hours, the average loss of BNP in samples without PPACK increased to 40%, while PPACK-containing samples showed 100% recovery compared with the initial BNP measurement. Initial values of BNP in samples at time 0 were, on average, 31% higher with PPACK added. These findings suggest that the addition of protease inhibitor immediately after centrifugation of plasma offers a valuable method of stabilizing patient samples and in increasing the shelf life of refrigerated samples without degradation of BNP. In addition, the data demonstrate the ability of PPACK to prevent BNP degradation, even after storage for 6 days at 4°C.

Table 15 below presents additional data for the patient samples analyzed above (Table 14) and demonstrates the ability of PPACK to prevent BNP degradation after storage for 6 hours at room temperature. Without PPACK at the respective time points shown in Tables 14 and 15, virtually no immunoreactive BNP remains. As in Table 14, the results are calculated as absolute recovery against the 1st run (time 0) measurement. KEY: 1 = patient number 1; 1p = patient number 1 with added PPACK protease inhibitor, etc.

**Table 15**

| Recovery of patient BNP with and without PPACK after storage for 6 hours at room temperature | | | |
|---|---|---|---|
| | BNP, pg/ml | BNP, pg/ml | Recovery vs 1st run (%) |
| Patient Sample* | | | |
| 1 | 65.4 | 48.8 | 74.6 |
| 1p | 88.9 | 83.4 | 93.8 |
| | | | |
| 4 | 171.3 | 123.5 | 72.1 |
| 4p | 197.8 | 205.6 | 104.0 |
| | | | |
| 5 | 109.6 | 83.9 | 76.6 |
| 5p | 151.7 | 147.2 | 97.0 |

| | | | |
|---|---|---|---|
| *: Insufficient sample volume to evaluate patient samples 2 and 3. | | | |

### EXAMPLE 8

In these analyses, two levels of synthetic BNP were spiked in different commercially available serum or plasma based diluents to compare BNP stability in the various diluents. All diluents were originally BNP free. The diluents used were as follows: diluent 1 was filtered horse serum; diluent 2 was heat-inactivated goat serum; diluent 3 was charcoal stripped defibrinated human plasma; diluent 4.was human defibrinated plasma (Irvine); diluent 5 was human defibrinated plasma (Seracon II); diluent 6 was true human serum; and diluent 7 was delipidated, stripped plasma (Seracon II).

The stability test was performed ~ 45 minutes after BNP was added to the diluents. The results are presented in Table 16. In Table 16, "low spike" indicates that BNP was added at a concentration of approximately 300 pg/ml, while "high spike" indicates that BNP was added at a concentration of approximately 3000 pg/ml. Despite the stabilizing effect of PPACK as demonstrated herein, BNP recovery was compromised, especially in diluent #7. These results elucidate the different activities of patient samples toward BNP degradation with and without protease inhibitors.

**Table 16**

| BNP sample | Diluent | Without inhibitor | With PPACK inhibitor (35 µg/ml) |
|---|---|---|---|
| | | pg/ml | pg/ml |
| low spike | diluent 1 | 231.3 | 291.5 |
| high spike | diluent 1 | 2423.0 | 2923.2 |
| low spike | diluent 2 | 240.4 | 271.7 |
| high spike | diluent 2 | 2350.0 | 2691.9 |
| low spike | diluent 3 | ** | 295.9 |
| high spike | diluent 3 | 5.8 | 2908.1 |
| low spike | diluent 4 | 45.7 | 407.3 |
| high spike | diluent 4 | 374.0 | 3801.2 |
| low spike | diluent 5 | 9.3 | 351.4 |
| high spike | diluent 5 | 71.6 | 3520.9 |
| low spike | diluent 6 | 59.0 | 367.2 |
| high spike | diluent 6 | 461.4 | 3457.3 |
| low spike | diluent 7 | 4.5 | 176.3 |
| high spike | diluent 7 | 26.9 | 2007.9 |

| | | | |
|---|---|---|---|
| **: below limit of detection | | | |

Table 17 shows that the dilution of BNP-positive plasma (i.e., plasma containing endogenous BNP) into normal plasma results in a different rate of BNP decay. This rate is specific to the plasma used as a diluent and, to a lesser degree, to the tested plasma. In Table 17, Plasma A, B and C represent samples received from the Cardiovascular Division, Brigham and Women's Hospital, Boston, MA. Normal plasma pool 1 was prepared in the laboratories of Bayer Corporation, Tarrytown, NY from in-house donors' plasma; plasma pool 2 is the same KCBB pool as described hereinabove; and plasma pools 3 and 4 were obtained from Intergen. "Dil" refers to "diluted".

**Table 17**

| | 1st run | After 24 hrs at 4°C | % recovery vs 1st run |
|---|---|---|---|
| | dose, pg/ml | dose, pg/ml | |
| Undiluted plasma A | 569.0 | 420.9 | 74.0 |
| 10-fold dil in normal plasma pool 1 | 70.4 | 53.8 | 76.4 |
| 10-fold dil in normal plasma pool 2 | 43.0 | 12.8 | 29.7 |
| 10-fold dil in normal plasma pool 3 | 64.3 | 47.7 | 74.2 |
| 10-fold dil in normal plasma pool 4 | 62.7 | 31.8 | 50.8 |
| | | | |
| Undiluted plasma B | 630.1 | 468.8 | 74.4 |
| 10-fold dil in normal plasma pool 1 | 73.9 | 49.8 | 67.3 |
| 10-fold dil in normal plasma pool 2 | 44.0 | 12.8 | 29.0 |
| 10-fold dil in normal plasma pool 3 | 61.6 | 45.7 | 74.3 |
| 10-fold dil in normal plasma pool 4 | 60.4 | 35.5 | 58.8 |
| | | | |
| Undiluted plasma C | 1504.0 | 1234.0 | 82.0 |
| 10-fold dil in normal plasma pool 1 | 152.8 | 111.3 | 72.8 |
| 10-fold dil in normal plasma pool 2 | 106.7 | 39.6 | 37.2 |
| 10-fold dil in normal plasma pool 3 | 147.8 | 116.0 | 78.5 |
| 10-fold dil in normal plasma pool 4 | 146.3 | 100.1 | 68.4 |

## Claims

1. A method of stabilizing brain natriuretic peptide (BNP) in a blood or plasma sample, comprising introducing a stabilizing amount of at least one stabilizing component selected from acetyl-leu-leu-arginal (leupeptin), N-(Nα-carbonyl-Arg-Val-Arg-al)Phe (antipain), H-D-Phe-Phe-Arg-chloromethylketone (PPACK), D-Phe-Pro-Arg-chloromethylketone (PPRACK), diisopropylfluorophosphate (DFP) and combinations thereof into the sample.

2. The method according to claim 1, further comprising one or more analogs, variants, or derivatives of the stabilizing components, wherein said analogs, variants and derivatives have BNP stabilizing function.

3. The method according to claim 1, wherein at least one stabilizing component is present in a collection vessel prior to collecting the blood or plasma sample.

4. The method according to claim 1, wherein at least one stabilizing component is introduced into the blood or plasma sample at the time of sample collection.

5. The method according to claim 1, wherein at least one stabilizing component is present in concentrated or lyophilized form.

6. A stabilized brain natriuretic peptide (BNP)-containing composition comprising BNP, and at least one component selected from acetyl-leu-leu-arginal (leupeptin), N-(Nα-carbonyl-Arg-Val-Arg-al)Phe (antipain), H-D-Phe-Phe-Arg-chloromethylketone (PPACK), D-Phe-Pro-Arg-chloromethylketone (PPRACK), diisopropylfluorophosphate (DFP) and combinations thereof.

7. The composition according to claim 6, wherein at least one component is present in concentrated or lyophilized form.

8. A control material for assaying samples containing, or suspected of containing, brain natriuretic peptide (BNP), comprising brain natriuretic peptide (BNP) and at least one BNP-stabilizing component selected from acetyl-leu-leu-arginal (leupeptin), N-(Nα-carbonyl-Arg-Val-Arg-al)Phe (antipain), H-D-Phe-Phe-Arg-chloromethylketone (PPACK), D-Phe-Pro-Arg-chloromethylketone (PPRACK), diisopropylfluorophosphate (DFP) and combinations thereof.

9. A kit for stabilizing brain natriuretic peptide (BNP) in blood based samples, comprising at least one container housing at least one component selected from acetyl-leu-leu-arginal (leupeptin), N-(Nα-carbonyl-Arg-Val-Arg-al)Phe (antipain), H-D-Phe-Phe-Arg-chloromethylketone (PPACK), D-Phe-Pro-Arg-chloromethylketone (PPRACK), diisopropylfluorophosphate (DFP) and combinations thereof, and, optionally, a dropper or similar device for dispensing the at least one component, a buffer for solubilizing the at least one component, synthetic or exogenous BNP, and instructions for use.

## Patentansprüche

1. Verfahren zur Stabilisierung des Brain-Natriuretic-Peptids (BNP) in einer Blut- oder Plasmaprobe, umfassend das Einführen einer stabilisierenden Menge zumindest einer stabilisierenden Komponente, die aus Acetyl-Leu-Leu-Arginal (Leupeptin), N-(Nα-Carbonyl-Arg-Val-Arg-al)Phe (Antipain), H-D-Phe-Phe-Arg-Chlormethylketon (PPACK), D-Phe-Pro-Arg-Chlormethylketon (PPRACK), Diisopropylfluorophosphat (DFP) und Kombinationen davon ausgewählt ist, in die Probe.

2. Verfahren nach Anspruch 1, weiters ein oder mehrere Analoga, Varianten oder Derivate der stabilisierenden Komponenten umfassend, worin die Analoga, Varianten und Derivate BNP-stabilisierende Funktion aufweisen.

3. Verfahren nach Anspruch 1, worin zumindest eine stabilisierende Komponente in einem Sammelbehälter vorhanden ist, bevor die Blut- oder Plasmaprobe entnommen wird.

4. Verfahren nach Anspruch 1, worin zum Zeitpunkt der Probenentnahme zumindest eine stabilisierende Komponente in die Blut- oder Plasmaprobe eingeführt wird.

5. Verfahren nach Anspruch 1, worin zumindest eine stabilisierende Komponente in konzentrierter oder gefriergetrockneter Form vorliegt.

6. Stabilisierte, Brain-Natriuretic-Peptid (BNP) enthaltende Zusammensetzung, die BNP und zumindest eine aus Acetyl-Leu-Leu-Arginal (Leupeptin), N-(Nα-Carbonyl-Arg-Val-Arg-al)Phe (Antipain), H-D-Phe-Phe-Arg-Chlormethylketon (PPACK), D-Phe-Pro-Arg-Chlormethylketon (PPRACK), Diisopropylfluorophosphat (DFP) und Kombinationen davon ausgewählte Komponente umfasst.

7. Zusammensetzung nach Anspruch 6, worin zumindest eine Komponente in konzentrierter oder gefriergetrockneter Form vorliegt.

8. Vergleichsmaterial zum Testen von Proben, die Brain-Natriuretic-Peptid (BNP) enthalten oder von denen vermutet wird, dass sie es enthalten, umfassend Brain-Natriuretic-Peptid (BNP) und zumindest eine BNP-stabilisierende Komponente, die aus Acetyl-Leu-Leu-Arginal (Leupeptin), N-(Nα-Carbonyl-Arg-Val-Arg-al)Phe (Antipain), H-D-Phe-Phe-Arg-Chlormethylketon (PPACK), D-Phe-Pro-Arg-Chlormethylketon (PPRACK), Diisopropylfluorophosphat (DFP) und Kombinationen davon ausgewählt ist.

9. Set zur Stabilisierung von Brain-Natriuretic-Peptid (BNP) in Proben auf Blutbasis, umfassend zumindest einen Behälter, in dem sich zumindest eine aus Acetyl-Leu-Leu-Arginal (Leupeptin), N-(Nα-Carbonyl-Arg-Val-Arg-al)Phe (Antipain), H-D-Phe-Phe-Arg-Chlormethylketon (PPACK), D-Phe-Pro-Arg-Chlormethylketon (PPRACK), Diisopropylfluorophosphat (DFP) und Kombinationen davon ausgewählte Komponente befindet, und gegebenenfalls eine Tropfpipette oder ähnliche Vorrichtung zur Abgabe der zumindest einen Komponente, einen Puffer zur Solubilisierung der zumindest einen Komponente, synthetisches oder exogenes BNP sowie Gebrauchsanweisungen.

## Revendications

1. Méthode de stabilisation du Brain Natriuretic Peptide (BNP) dans un échantillon de sang ou de plasma, comprenant l'introduction dans l'échantillon d'une quantité stabilisante d'au moins un composant stabilisant choisi entre acétyl-leu-leu-arginal (leupeptine), N-(Nα-carbonyl-Arg-Val-Arg-al)Phe (antidouleur), H-D-Phe-Phe-Arg-chloro-méthylcétone (PPACK), D-Phe-Pro-Arg-chlorométhylcétone (PPRACK), diisopropylfluorophosphate (DFP) et des combinaisons de ces composants.

2. Méthode suivant la revendication 1, comprenant en outre un ou plusieurs analogues, variants ou dérivés des composants stabilisants, ces analogues, variants et dérivés ayant une fonction de stabilisation du BNP.

3. Méthode suivant la revendication 1, dans laquelle au moins un composant stabilisant est présent dans un récipient de prélèvement avant que l'échantillon de sang ou de plasma y soit recueilli.

4. Méthode suivant la revendication 1, dans laquelle au moins un composant stabilisant est introduit dans l'échantillon de sang ou de plasma au moment du prélèvement de l'échantillon.

5. Méthode suivant la revendication 1, dans laquelle au moins un composant stabilisant est présent sous une forme concentrée ou lyophilisée.

6. Composition contenant du Brain Natriuretic Peptide (BNP) stabilisé, comprenant du BNP et au moins un composant choisi entre acétyl-leu-leu-arginal (leupeptine), N-(Na-carbonyl-Arg-Val-Arg-al)Phe (antidouleur), H-D-Phe-Phe-Arg-chlorométhylcétone (PPACK), D-Phe-Pro-Arg-chloro-méthylcétone (PPRACK), diisopropylfluorophosphate (DFP) et des combinaisons de ces composants.

7. Composition suivant la revendication 6, dans laquelle au moins un composant est présent sous une forme concentrée ou lyophilisée.

8. Matière témoin destinée à l'analyse d'échantillons contenant du Brain Natriuretic Peptide (BNP) ou suspectés d'en contenir, comprenant du Brain Natriuretic Peptide (BNP) et au moins un composant stabilisant le BNP, choisi entre acétyl-leu-leu-arginal (leupeptine), N-(Nα-carbonyl-Arg-Val-Arg-al)Phe (antidouleur), H-D-Phe-Phe-Arg-chlorométhylcétone (PPACK), D-Phe-Pro-Arg-chlorométhylcétone (PPRACK), diisopropylfluorophosphate (DFP) et des combinaisons de ces composants.

9. Kit destiné à la stabilisation du Brain Natriuretic Peptide (BNP) dans des échantillons à base de sang, comprenant au moins un récipient renfermant au moins un composant choisi entre acétyl-leu-leu-arginal (leupeptine), N-(Nα-carbonyl-Arg-Val-Arg-al)Phe (antidouleur), H-D-Phe-Phe-Arg-chlorométhylcétone (PPACK), D-Phe-Pro-Arg-chloro-méthylcétone (PPRACK), diisopropylfluorophosphate (DFP) et des combinaisons de ces composants et, à titre facultatif, un compte-gouttes ou un dispositif similaire permettant de distribuer le ou les composants, un tampon de solubilisation du ou des composants, du BNP synthétique ou exogène et un mode d'emploi.
